# EUROPEAN PATENT SPECIFICATION

(11) **EP 3 836 263 B1**
(45) Date of publication and mention of the grant of the patent: **02.09.2026**
(21) Application number: 20179598.6
(22) Date of filing: 12.06.2020
(51) Int. Cl.: H01M 4/52, H01M 4/583, H01M 10/0525, H01M 10/0567, H01M 10/0568, H01M 10/0569

(54) **ELECTROLYTE SOLUTION FOR LITHIUM SECONDARY BATTERIES AND LITHIUM SECONDARY BATTERY INCLUDING THE SAME**
ELEKTROLYTLÖSUNG FÜR LITHIUMSEKUNDÄRBATTERIEN UND LITHIUMSEKUNDÄRBATTERIE DAMIT
SOLUTION D'ÉLECTROLYTE POUR BATTERIES SECONDAIRES AU LITHIUM ET BATTERIE SECONDAIRE AU LITHIUM L'INCLUANT

(30) Priority: 13.12.2019 KR 20190167265
(43) Date of publication of application: 16.06.2021
(73) Proprietor: HYUNDAI MOTOR COMPANY, Seoul 06797 (KR); Kia Corporation, Seocho-gu Seoul 06797 (KR)
(72) Inventor: LEE, Yoon Sung, 06797 Seoul (KR); KIM, Ik Kyu, 06797 Seoul (KR); OH, Seung Min, 06797 Seoul (KR); LEE, Young Woo, 06797 Seoul (KR); KIM, Dong Jun, 06797 Seoul (KR); BAN, Sung Ho, 06797 Seoul (KR); LIM, Sung Hoon, 06797 Seoul (KR)
(74) Representative: Isarpatent

(56) References cited:
- PETIBON R ET AL: "Evaluation of phenyl carbonates as electrolyte additives in lithium-ion batteries", JOURNAL OF POWER SOURCES, ELSEVIER SA, CH, vol. 287, 4 April 2015 (2015-04-04), pages 184 - 195, XP029158087, ISSN: 0378-7753, DOI: 10.1016/J.JPOWSOUR.2015.04.012
- SHENG SHUI ZHANG: "A review on electrolyte additives for lithium-ion batteries", JOURNAL OF POWER SOURCES, vol. 162, no. 2, 1 November 2006 (2006-11-01), CH, pages 1379 - 1394, XP055557116, ISSN: 0378-7753, DOI: 10.1016/j.jpowsour.2006.07.074

## Description

### TECHNICAL FIELD

The present invention relates to an electrolyte solution for lithium secondary batteries and a lithium secondary battery including the same. In particular, the electrolyte solution for lithium secondary batteries may increase the lifetime of the lithium secondary batteries.

### BACKGROUND OF THE INVENTION

A lithium secondary battery is an energy storage device that includes a positive electrode for supplying lithium and a negative electrode for receiving lithium during charging, an electrolyte serving as a medium for transferring a lithium ion, and a separator for separating the positive electrode and the negative electrode from each other. The lithium secondary battery generates electrical energy and stores the same through a change in chemical potential when the lithium ion is intercalated or de-intercalated on the positive electrode or the negative electrode.

Such a lithium secondary battery has mainly been used in portable electronic devices, but has recently come to be used as energy storage means for electric vehicles (EVs) and hybrid electric vehicles (HEVs) in response to recent commercialization of electric vehicles (EVs) and hybrid electric vehicles (HEVs).

Meanwhile, research on increasing the energy density of the lithium secondary battery has been conducted in order to increase the driving distance of electric vehicles, and the energy density of lithium secondary batteries has been increased by increasing the capacity of the positive electrode.

The increase in the capacity of the positive electrode can be achieved through Ni enrichment, which is a method of increasing the Ni content of Ni-Co-Mn-based oxide constituting a positive-electrode active material, or can be achieved by increasing the positive-electrode charging voltage.

However, Ni-enriched Ni-Co-Mn-based oxides have high interfacial reactivity and an unstable crystal structure, disadvantageously accelerating deterioration during cycles and making it difficult to secure long-life performance.

Petibon R. et Al, JOURNAL OF POWER SOURCES, vol. 287, pages 184-195 teaches phenyl carbonates as electrolyte additives in lithium ion batteries.

The above information disclosed in this Background section is provided only for enhancement of understanding of the background of the invention and therefore it may contain information that does not form the prior art that is already known in this country to a person of ordinary skill in the art.

### SUMMARY OF THE INVENTION

In preferred aspect, provided are, *inter alia,* an electrolyte solution for lithium secondary batteries which may increase the lifetime of the lithium secondary batteries and a lithium secondary battery including the electrolyte.

In an aspect, provided is an electrolyte solution for lithium secondary batteries including a lithium salt, a solvent and allyl (4-(1,3-bis(4-(((allyloxy)carbonyl)oxy)-5-(tert-butyl)-2-methylphenyl)butyl)-2-(tert-butyl)-5-methylphenyl) represented by the following Formula 1.

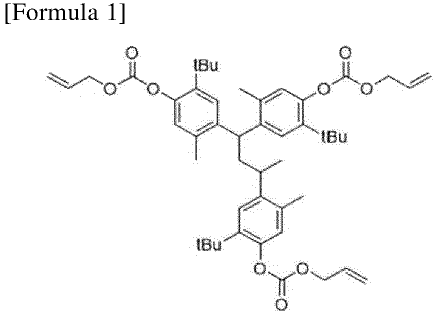

The electrolyte solution may suitably include the llyl (4-(1,3-bis(4-(((allyloxy)carbonyl)oxy)-5-(tert-butyl)-2-methylphenyl)butyl)-2-(tert-butyl)-5-methylphenyl) in an amount of about 3.0% by weight or less with respect to the total weight of the electrolyte solution.

Preferably, the electrolyte solution may suitably include the llyl (4-(1,3-bis(4-(((allyloxy)carbonyl)oxy)-5-(tert-butyl)-2-methylphenyl)butyl)-2-(tert-butyl)-5-methylphenyl) in an amount of about 0.5 to 2.0% by weight with respect to the total weight of the electrolyte solution.

The lithium salt may suitably include one or more selected from the group consisting of LiPF₆, LiBF₄, LiClO₄, LiCl, LiBr, LiI, LiB₁₀Cl₁₀, LiCF₃SO₃, LiCF₃CO₂, LiAsF₆, LiSbF₆, LiAlCl₄, CH₃SO₃Li, CF₃SO₃Li, LiN(SO₂C₂F₅)₂, Li(CF₃SO₂)₂N, LiC₄F₉SO₃, LiB(C₆H₅)₄, Li(SO₂F)₂N (LiFSI) and (CF₃SO₂)₂NLi.

The solvent may suitably include one or more selected from the group consisting of carbonate solvents, ester solvents, ether solvents and ketone solvents. Other solvents also may be suitable.

The electrolyte solution for lithium secondary batteries may further include a positive-electrode additive, for example wherein the positive-electrode additive is LiPO₂F₂.

In another aspect, provided is a lithium secondary battery including the electrolyte solution described above. In addition, the lithium secondary battery may further include a positive electrode including a positive-electrode active material containing Ni, Co and Mn, a negative electrode including a carbon (C)-based negative-electrode active material, and a separator interposed between the positive electrode and the negative electrode.

The lithium secondary battery may be a discharge retention of 93% or more, which is measured after 200 cycles, each cycle including 0.5C cc/cv charging and 0.5C cc/cv discharging at 2.5 to 4.2V (cut-off) and at a temperature of 45 °C.

Vehicles are also provided that comprises a lithium secondary battery as disclosed herein.

### Other aspects of the invention

### BRIEF DESCRIPTION OF THE DRAWINGS

The above and other objects, features and other advantages of the present invention will be more clearly understood from the following detailed description taken in conjunction with the accompanying drawings, in which:
FIG. 1 is a graph showing the evaluation results of characteristics after addition of additives in Experiment 1 according an exemplary embodiment of the present invention.
FIG. 2 is a graph showing the evaluation results of characteristics after addition of additives in Experiment 2 according an exemplary embodiment of the present invention.
FIG. 3 is a graph showing the evaluation results of characteristics after addition of additives in Experiment 3 according an exemplary embodiment of the present invention.

### DETAILED DESCRIPTION

Reference will now be made in detail to the preferred embodiments of the present invention, examples of which are illustrated in the accompanying drawings. However, the present invention is not limited to the embodiments, and may be implemented in various forms. The embodiments are provided only to fully illustrate the present invention and to completely inform those having ordinary knowledge in the art of the scope of the present invention.

The terminology used herein is for the purpose of describing particular exemplary embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

Unless specifically stated or obvious from context, as used herein, the term "about" is understood as within a range of normal tolerance in the art, for example within 2 standard deviations of the mean. "About" can be understood as within 10%, 9%, 8%, 7%, 6%, 5%, 4%, 3%, 2%, 1%, 0.5%, 0.1%, 0.05%, or 0.01% of the stated value. Unless otherwise clear from the context, all numerical values provided herein are modified by the term "about."

It is understood that the term "vehicle" or "vehicular" or other similar term as used herein is inclusive of motor vehicles in general such as passenger automobiles including sports utility vehicles (SUV), buses, trucks, various commercial vehicles, watercraft including a variety of boats and ships, aircraft, and the like, and includes hybrid vehicles, electric vehicles, combustion, plug-in hybrid electric vehicles, hydrogen-powered vehicles and other alternative fuel vehicles (e.g. fuels derived from resources other than petroleum).

It is also understood that the term "solution" as used herein includes dispersions and other fluid admixtures in addition to true solutions.

In an aspect, an electrolyte solution for lithium secondary batteries may be a material constituting an electrolyte applicable to lithium secondary batteries and includes a lithium salt, a solvent and a negative-electrode additive. In addition, the electrolyte solution may further include a positive-electrode additive.

The lithium salt may be one or more selected from the group consisting of LiPF₆, LiBF₄, LiClO₄, LiCl, LiBr, LiI, LiB₁₀Cl₁₀, LiCF₃SO₃, LiCF₃CO₂, LiAsF₆, LiSbF₆, LiAlCl₄, CH₃SO₃Li, CF₃SO₃Li, LiN(SO₂C₂F₅)₂, Li(CF₃SO₂)₂N, LiC₄F₉SO₃, LiB(C₆H₅)₄, Li(SO₂F)₂N (LiFSI) and (CF₃SO₂)₂NLi.

In this case, the electrolyte solution may suitably include the lithium salt in a concentration of about 0.1 to 1.2 M in the electrolyte solution.

The solvent may suitably include one or more selected from the group consisting of carbonate solvents, ester solvents, ether solvents and ketone solvents.

For instance, the carbonate solvent may suitably include dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), butylene carbonate (BC), fluoroethylene carbonate (FEC), vinylene carbonate (VC) or the like. In addition, the carbonate solvent may be an ester solvent such as γ-butyrolactone (GBL), n-methyl acetate, n-ethyl acetate or n-propyl acetate, or an ether solvent such as dibutyl ether, but is not limited thereto.

In addition, the solvent may further include an aromatic hydrocarbon-based organic solvent. Specific examples of the aromatic hydrocarbon-based organic solvent may suitably include benzene, fluorobenzene, bromobenzene, chlorobenzene, cyclohexylbenzene, isopropylbenzene, n-butylbenzene, octylbenzene, toluene, xylene, mesitylene, and the like, and this solvent may be used alone or in combination.

In addition, LiPO₂F₂ may be used as the positive-electrode additive.

Meanwhile, allyl (4-(1,3-bis(4-(((allyloxy)carbonyl)oxy)-5-(tert-butyl)-2-methylphenyl)butyl)-2-(tert-butyl)-5-methylphenyl), represented by the following Formula 1 may be used as the negative-electrode additive added to the electrolyte solution according to the embodiment of the present invention.

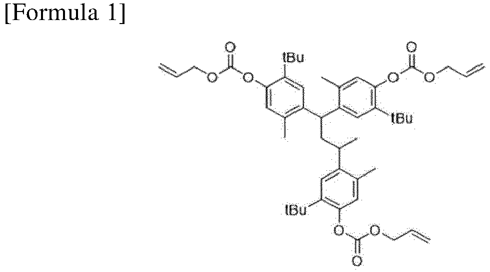

In this case, the negative-electrode additive may improve the low-resistance characteristics and increase the lifespan by forming a solid electrolyte interphase (SEI) on the negative electrode. Preferably, the negative-electrode additive may be added in an amount of about 3.0% by weight or less, or particularly, in an amount of about 0.5 to 2.0% by weight, with respect to the total weight of the electrolyte solution.

When the amount of the negative-electrode additive added is greater than about 3.0% by weight, the coating film of the negative electrode may be excessively formed, disadvantageously resulting in high cell resistance and thus decreased cell power. When the amount of the negative-electrode additive is less than about 0.5% by weight, there is a problem in that the SEI, which is a protective film of the negative electrode, may be insufficiently formed and thus the lifespan of the cell may be greatly reduced. When the amount of the negative-electrode additive is greater than about 2.0% by weight, there occurs a problem in that cell power required for vehicles may decreased.

In another aspect, the lithium secondary battery may include a positive electrode, a negative electrode and a separator, and the electrolyte solution as described herein.

The positive electrode may suitably include an NCM-based positive-electrode active material containing Ni, Co and Mn. In particular, the positive-electrode active material included in the positive electrode in this embodiment may contain only an NCM-based positive-electrode active material containing Ni in an amount of 60% by weight or greater based on the total weight of the NCM-based positive electrode active material.

In addition, the negative electrode may suitably include a carbon (C)-based negative-electrode active material alone or may include a carbon (C)-based negative-electrode active material.

The carbon (C)-based negative-electrode active material may suitably include at least one material selected from the group consisting of artificial graphite, natural graphite, graphitized carbon fiber, graphitized mesocarbon microbeads, fullerene and amorphous carbon.

Meanwhile, the positive electrode and the negative electrode may be produced by mixing each of active materials with a conductive material, a binder and a solvent to prepare an electrode slurry, and then directly coating a current collector with the electrode slurry, followed by drying. In this case, aluminum (Al) may be used as the current collector, but the present invention is not limited thereto. Since such an electrode production method is well known in the art, a detailed description thereof will be omitted.

The binder may facilitate adhesion between particles of each active material or adhesion thereof to the current collector. For example, the binder may suitably include polyvinyl alcohol, carboxymethyl cellulose, hydroxypropyl cellulose, diacetyl cellulose, polyvinyl chloride, carboxylated polyvinylchloride, polyvinylfluoride, an ethylene-oxide-containing polymer, polyvinylpyrrolidone, polyurethane, polytetrafluoroethylene, polyvinylidene fluoride, polyethylene, polypropylene, styrenebutadiene rubber, acrylated styrene butadiene rubber, an epoxy resin, nylon, or the like, but is not limited thereto.

In addition, the conductive material may impart conductivity to the electrode, and any one can be used as long as it is an electrically conductive material that does not cause a chemical change in the battery to be produced, and examples thereof include natural graphite, artificial graphite, carbon black, acetylene black, Ketjen black, carbon fibers, metal powders, such as copper, nickel, aluminum and silver powders, metal fibers and the like. In addition, a conductive material such as a polyphenylene derivative may be used alone or in combination thereof.

The separator prevents a short circuit between the positive electrode and the negative electrode, and provides a passage for lithium ions. Such a separator may suitably include one or more selected from polyolefin-based polymer membranes such as polypropylene, polyethylene, polyethylene/polypropylene, polyethylene/polypropylene/polyethylene and polypropylene/polyethylene/polypropylene, and multiple membranes, microporous films, woven fabrics and nonwoven fabrics thereof. In addition, a porous polyolefin film coated with a resin having excellent stability may be used.

### EXAMPLE

Hereinafter, the present invention will be described with reference to Examples and Comparative Examples according to the present invention.

### <Experiment 1> Experiment of characteristics depending on type of negative-electrode additive

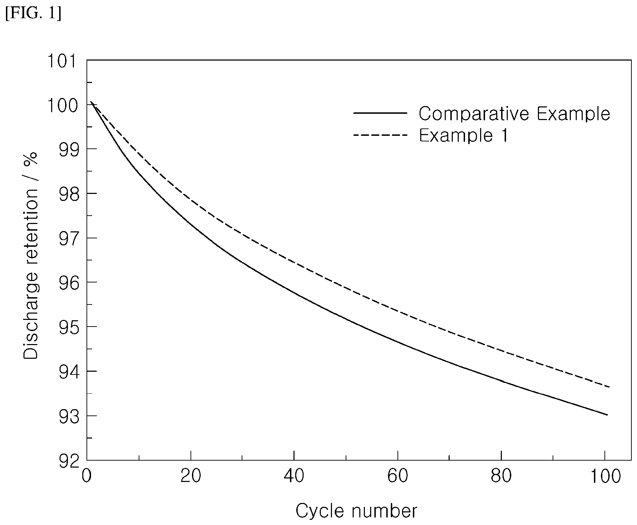

In order to determine various characteristics depending on the type of the negative-electrode additive added to the electrolyte solution, ion conductivity, initial cell resistance, high-temperature durability and high-rate characteristics were measured while changing the type of the negative-electrode additive as shown in Table 1 below, and the result is shown in Table 2 and FIG. 1.

At this time, the lithium salts used to prepare the electrolyte solution were 0.5M LiPF₆ and 0.5M LiFSI, and the solvent herein used was a mixture of ethylene carbonate (EC), ethyl methyl carbonate (EMC) and diethyl carbonate (DEC) present at a volume ratio of 25:45:30. In addition, LiPO₂F₂ was used as a positive-electrode additive.

NCMN811 was used as the positive electrode and graphite was used as the negative electrode.

At this time, the measurement conditions of ion conductivity, initial cell resistance, high-temperature durability and high-rate characteristics are as follows.
- Ion conductivity: measured at room temperature (25°C)
- Initial cell resistance: cell DC-IR measured after formation
- High-temperature durability: charging at 0.5C cc/cv and then discharging at 0.5C cc, at 2.5 to 4.2V (cut-off) and at a temperature of 45°C during each cycle
- High-rate characteristics: capacity expression value determined while increasing discharge after charging only 0.1C cc/cv during every cycle

**Table 1**

| Item | Lithium salt (M) | | Solvent (weight ratio) | | | Positive-electrode additive (wt%) | Negative-electrode additive (wt%) | |
|---|---|---|---|---|---|---|---|---|
| | LiPF₆ | LiFSI | EC | EMC | DEC | LiPO₂F₂ | VC | [Formula 1] Additive |
| Comparative Example | 0.5 | 0.5 | 25 | 45 | 30 | 1 | 2 | - |
| Example 1 | 0.5 | 0.5 | 25 | 45 | 30 | 1 | - | 2 |

**Table 2**

| Item | Ionic conductivity (mS/cm) | Initial cell resistance (%) | High-temperature durability (%)@ 100cyc | High-rate characteristics 2C(%) |
|---|---|---|---|---|
| Comparative Example | 8.21 | 100 | 93 | 48.6 |
| Example 1 | 8.28 | 97.6 | 93.7 | 54.7 |

As shown in Table 2 and FIG. 1, when allyl(4-(1,3-bis(4-(((allyloxy)carbonyl)oxy)-5-(tert-butyl)-2-methylphenyl)butyl)-2-(tert-butyl)-5-methylphenyl), represented by Formula 1, was used as a negative-electrode additive, improvement in ion conductivity, high-temperature durability and high-rate characteristics was obtained as compared to Comparative Example using a conventional general additive VC as a negative-electrode additive under the same conditions. In particular, improved high-rate characteristics along with excellent lifetime in the same content satisfied the performance suitable for vehicle batteries.

### <Experiment 2> Experiment on high-rate characteristics of negative-electrode additive

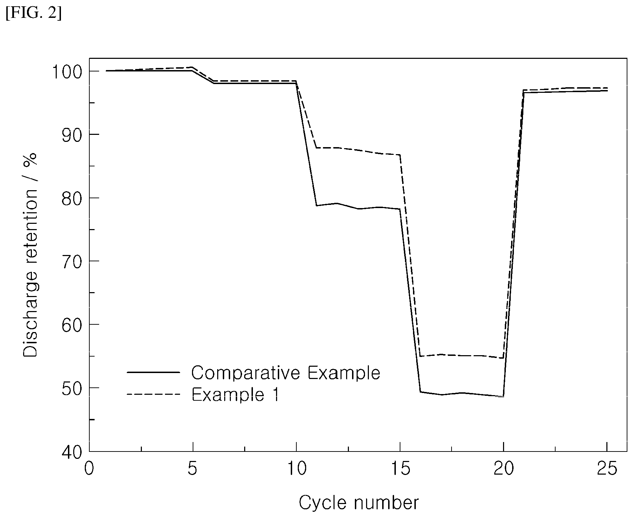

Charging and discharging were performed at 0.5C, 1.0C, 2.0C and 0.1C on Comparative Example and Example 1 of <Experiment 1>, respectively, the corresponding capacity expression values were determined, and the results are shown in FIG. 2.

As shown in FIG. 2, when allyl (4-(1,3-bis(4-(((allyloxy)carbonyl)oxy)-5-(tert-butyl)-2-methylphenyl)butyl)-2-(tert-butyl)-5-methylphenyl) represented by Formula 1 was used as a negative-electrode additive, improved high-rate characteristics were obtained compared to Comparative Example using a conventional general additive VC as a negative-electrode additive under the same conditions. This means that the improved high-rate characteristics can be based on excellent ion conductivity.

### <Experiment 3> Experiment of characteristics depending on content of negative-electrode additive

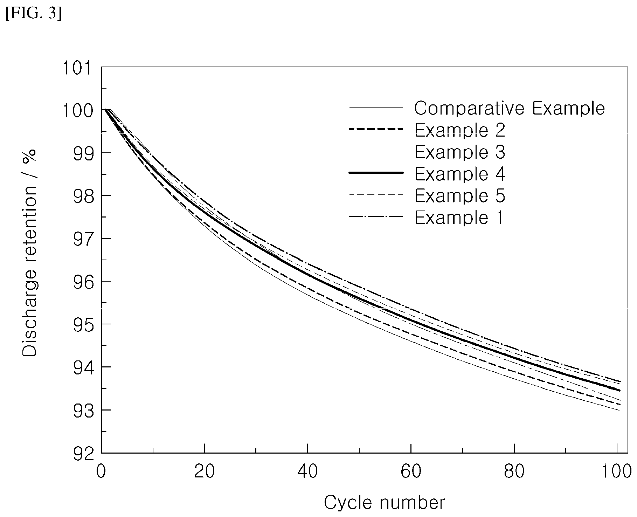

In order to determine various characteristics depending on the type of the negative-electrode additive that is added to the electrolyte solution, ion conductivity, initial cell resistance, high-temperature durability and high-rate characteristics were measured while changing the type of the negative-electrode additive as shown in Table 3 below, and the result is shown in Table 4 and FIG. 3. In this case, other conditions and measurement methods are the same as in <Experiment 1>.

**Table 3**

| Item | Lithium salt (M) | | Solvent (Weight ratio) | | | Positive-electrode additive (wt%) | Negative-electrode additive (wt%) | |
|---|---|---|---|---|---|---|---|---|
| | LiPF₆ | LiFSI | EC | EMC | DEC | LiPO₂F₂ | VC | [Formula 1] Additive |
| Comparative Example | 0.5 | 0.5 | 25 | 45 | 30 | 1 | 2 | - |
| Example 2 | 0.5 | 0.5 | 25 | 45 | 30 | 1 | - | 0.2 |
| Example 3 | 0.5 | 0.5 | 25 | 45 | 30 | 1 | - | 0.5 |
| Example 4 | 0.5 | 0.5 | 25 | 45 | 30 | 1 | - | 1 |
| Example 5 | 0.5 | 0.5 | 25 | 45 | 30 | 1 | - | 1.5 |
| Example 1 | 0.5 | 0.5 | 25 | 45 | 30 | 1 | - | 2 |

**Table 4**

| Item | Ionic conductivity (mS/cm) | Initial cell resistance (%) | High-temperature durability (%)@ 100cyc |
|---|---|---|---|
| Comparative Example | 8.21 | 100 | 93 |
| Example 2 | 8.73 | 90.5 | 93.1 |
| Example 3 | 8.56 | 92.1 | 93.2 |
| Example 4 | 8.41 | 94.5 | 93.4 |
| Example 5 | 8.36 | 96.4 | 93.6 |
| Example 1 | 8.28 | 97.6 | 93.7 |

As shown in Table 4 and FIG. 2, Examples 1 to 5 including allyl(4-(1,3-bis(4-(((allyloxy)carbonyl)oxy)-5-(tert-butyl)-2-methylphenyl)butyl)-2-(tert-butyl)-5-methylphenyl) represented by Formula 1 as a negative-electrode additive had improved ion conductivity, and similar or better high-temperature durability, compared to Comparative Example using a conventional general additive VC as a negative-electrode additive under the same conditions.

In particular, Example 2, in which the amount of allyl(4-(1,3-bis(4-(((allyloxy)carbonyl)oxy)-5-(tert-butyl)-2-methylphenyl)butyl)-2-(tert-butyl)-5-methylphenyl) that was added was 0.2% by weight, had similar high-temperature durability to Comparative Example using a conventional general additive VC as a negative-electrode additive under the same conditions. However, the high-temperature durability thereof was improved as the amount of the negative-electrode additive added increases.

Therefore, the negative-electrode additive is preferably added in an amount of about 0.5 to 2.0% by weight with respect to the total weight of the electrolyte solution.

According to various exemplary embodiments of the present invention, by adding an additive for forming an SEI film on a negative electrode to an electrolyte solution, the effect of increasing the long-term lifespan of lithium secondary batteries can be expected.

Although the exemplary embodiments of the present invention have been disclosed for illustrative purposes, those skilled in the art will appreciate that various modifications, additions and substitutions are possible, without departing from the scope of the invention as disclosed in the accompanying claims.

## Claims

1. An electrolyte solution for lithium secondary batteries comprising:
a lithium salt;
a solvent; and
allyl(4-(1,3-bis(4-(((allyloxy)carbonyl)oxy)-5-(tert-butyl)-2-methylphenyl)butyl)-2-(tert-butyl)-5-methylphenyl) represented by the following Formula 1.

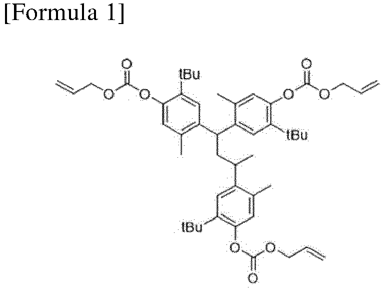

2. The electrolyte solution for lithium secondary batteries according to claim 1, wherein the electrolyte solution comprises the allyl(4-(1,3-bis(4-(((allyloxy)carbonyl)oxy)-5-(tert-butyl)-2-methylphenyl)butyl)-2-(tert-butyl)-5-methylphenyl) in an amount of about 3.0% by weight or less with respect to the total weight of the electrolyte solution.

3. The electrolyte solution for lithium secondary batteries according to claim 2, wherein the electrolyte solution comprises the allyl(4-(1,3-bis(4-(((allyloxy)carbonyl)oxy)-5-(tert-butyl)-2-methylphenyl)butyl)-2-(tert-butyl)-5-methylphenyl) in an amount of about 0.5 to 2.0% by weight with respect to the total weight of the electrolyte solution.

4. The electrolyte solution for lithium secondary batteries according to claim 1, wherein the lithium salt comprises one or more selected from the group consisting of LiPF₆, LiBF₄, LiClO₄, LiCl, LiBr, LiI, LiB₁₀Cl₁₀, LiCF₃SO₃, LiCF₃CO₂, LiAsF₆, LiSbF₆, LiAlCl₄, CH₃SO₃Li, CF₃SO₃Li, LiN(SO₂C₂F₅)₂, Li(CF₃SO₂)₂N, LiC₄F₉SO₃, LiB(C₆H₅)₄, Li(SO₂F)₂N (LiFSI) and (CF₃SO₂)₂NLi.

5. The electrolyte solution for lithium secondary batteries according to claim 1, wherein the solvent comprises one or more selected from the group consisting of carbonate solvents, ester solvents, ether solvents and ketone solvents.

6. The electrolyte solution for lithium secondary batteries according to claim 1, further comprising a positive-electrode additive,
wherein the positive-electrode additive comprises LiPO₂F₂.

7. A lithium secondary battery comprising the electrolyte solution according to claim 1.

8. The lithium secondary battery according to claim 7, further comprising:
a positive electrode comprising a positive-electrode active material containing Ni, Co and Mn;
a negative electrode comprising a carbon (C)-based negative-electrode active material; and
a separator interposed between the positive electrode and the negative electrode.

9. The lithium secondary battery according to claim 7, wherein the lithium secondary battery has a discharge retention of about 93% or greater, measured after 200 cycles, each cycle including 0.5C cc/cv charging and 0.5C cc/cv discharging at 2.5 to 4.2V (cut-off) and at a temperature of 45 °C.

10. A vehicle comprising the lithium secondary battery of claim 7.

## Patentansprüche

1. Elektrolytlösung für Lithiumsekundärbatterien, umfassend:
ein Lithiumsalz;
ein Lösungsmittel; und
Allyl(4-(1,3-bis(4-(((allyloxy)carbonyl)oxy)-5-(tert-butyl)-2-methylphenyl)butyl)-2-(tert-butyl)-5-methylphenyl), dargestellt durch die folgende Formel 1.

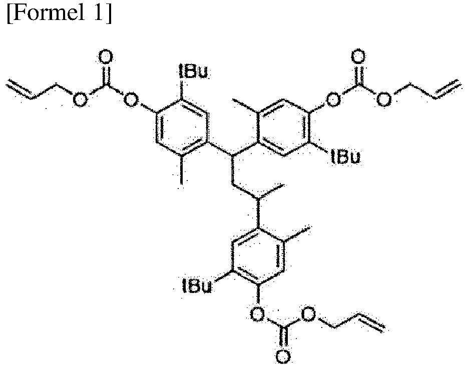

2. Elektrolytlösung für Lithiumsekundärbatterien nach Anspruch 1, wobei die Elektrolytlösung das Allyl(4-(1,3-bis(4-(((allyloxy)carbonyl)oxy)-5-(tert-butyl)-2-methylphenyl)butyl)-2-(tert-butyl)-5-methylphenyl) in einer Menge von etwa 3,0 Gew.-% oder weniger in Bezug auf das Gesamtgewicht der Elektrolytlösung umfasst.

3. Elektrolytlösung für Lithiumsekundärbatterien nach Anspruch 2, wobei die Elektrolytlösung das Allyl(4-(1,3-bis(4-(((allyloxy)carbonyl)oxy)-5-(tert-butyl)-2-methylphenyl)butyl)-2-(tert-butyl)-5-methylphenyl) in einer Menge von etwa 0,5 bis 2,0 Gew.-% in Bezug auf das Gesamtgewicht der Elektrolytlösung umfasst.

4. Elektrolytlösung für Lithiumsekundärbatterien nach Anspruch 1, wobei das Lithiumsalz eines oder mehrere, ausgewählt aus der Gruppe bestehend aus LiPF₆, LiBF₄, LiClO₄, LiCl, LiBr, LiI, LiB₁₀Cl₁₀, LiCF₃SO₃, LiCF₃CO₂, LiAsF₆, LiSbF₆, LiAlCl₄, CH₃SO₃Li, CF₃SO₃Li, LiN(SO₂C₂F₅)₂, Li(CF₃SO₂)₂N, LiC₄F₉SO₃, LiB(C₆H₅)₄, Li(SO₂F)₂N (LiFSI) und (CF₃SO₂)₂NLi, umfasst.

5. Elektrolytlösung für Lithiumsekundärbatterien nach Anspruch 1, wobei das Lösungsmittel eines oder mehrere, ausgewählt aus der Gruppe bestehend aus Carbonat-Lösungsmitteln, Ester-Lösungsmitteln, Ether-Lösungsmitteln und Keton-Lösungsmitteln, umfasst.

6. Elektrolytlösung für Lithiumsekundärbatterien nach Anspruch 1, weiterhin umfassend ein Positivelektroden-Additiv,
wobei das Positivelektroden-Additiv LiPO₂F₂ umfasst.

7. Lithiumsekundärbatterie, umfassend die Elektrolytlösung nach Anspruch 1.

8. Lithiumsekundärbatterie nach Anspruch 7, weiterhin umfassend:
eine positive Elektrode, umfassend ein Ni, Co und Mn enthaltendes Positivelektroden-Aktivmaterial;
eine negative Elektrode, umfassend ein Negativelektroden-Aktivmaterial auf Basis von Kohlenstoff (C); und
einen Separator, eingefügt zwischen der positiven Elektrode und der negativen Elektrode.

9. Lithiumsekundärbatterie nach Anspruch 7, wobei die Lithiumsekundärbatterie eine Retention der Entladung von etwa 93 % oder größer aufweist, gemessen nach 200 Zyklen, wobei jeder Zyklus Laden mit 0,5C, CC/CV, und Entladen mit 0,5C, CC/CV, bei 2,5 bis 4,2 V (Obergrenze) und bei einer Temperatur von 45 °C umfasst.

10. Fahrzeug, umfassend eine Lithiumsekundärbatterie nach Anspruch 7.

## Revendications

1. Solution d'électrolyte pour batteries secondaires au lithium, comprenant :
un sel de lithium ;
un solvant ; et
de l'allyl(4-(1,3-bis(4-(((allyloxy)carbonyl)oxy)-5-(tert-butyl)-2-méthylphényl)butyl)-2-(tert-butyl)-5-méthylphényle) représenté par la Formule 1 suivante.

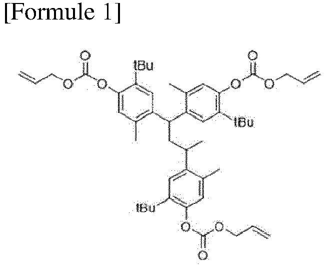

2. Solution d'électrolyte pour batteries secondaires au lithium selon la revendication 1, la solution d'électrolyte comprenant l'allyl(4-(1,3-bis(4-(((allyloxy)carbonyl)oxy)-5-(tert-butyl)-2-méthylphényl)butyl)-2-(tert-butyl)-5-méthylphényle) en une quantité d'environ 3,0 % en poids ou moins par rapport au poids total de la solution d'électrolyte.

3. Solution d'électrolyte pour batteries secondaires au lithium selon la revendication 2, la solution d'électrolyte comprenant l'allyl(4-(1,3-bis(4-(((allyloxy)carbonyl)oxy)-5-(tert-butyl)-2-méthylphényl)butyl)-2-(tert-butyl)-5-méthylphényle) en une quantité d'environ 0,5 à 2,0 % en poids par rapport au poids total de la solution d'électrolyte.

4. Solution d'électrolyte pour batteries secondaires au lithium selon la revendication 1, le sel de lithium comprenant un ou plusieurs éléments sélectionnés dans le groupe constitué par LiPF₆, LiBF₄, LiClO₄, LiCl, LiBr, LiI, LiB₁₀Cl₁₀, LiCF₃SO₃, LiCF₃CO₂, LiAsF₆, LiSbF₆, LiAlCl₄, CH₃SO₃Li, CF₃SO₃Li, LiN(SO₂C₂F₅)₂, Li(CF₃SO₂)₂N, LiC₄F₉SO₃, LiB(C₆H₅)₄, Li(SO₂F)₂N (LiFSI) et (CF₃SO₂)₂NLi.

5. Solution d'électrolyte pour batteries secondaires au lithium selon la revendication 1, le solvant comprenant un ou plusieurs sélectionnés dans le groupe constitué par les solvants carbonates, les solvants esters, les solvants éthers et les solvants cétones.

6. Solution d'électrolyte pour batteries secondaires au lithium selon la revendication 1, comprenant en outre un additif d'électrode positive,
l'additif d'électrode positive comprenant du LiPO₂F₂.

7. Batterie secondaire au lithium comprenant la solution d'électrolyte selon la revendication 1.

8. Batterie secondaire au lithium selon la revendication 7, comprenant en outre :
une électrode positive comprenant un matériau actif d'électrode positive contenant du Ni, du Co et du Mn ;
une électrode négative comprenant un matériau actif d'électrode négative à base de carbone (C) ; et
un séparateur interposé entre l'électrode positive et l'électrode négative.

9. Batterie secondaire au lithium selon la revendication 7, la batterie secondaire au lithium présentant une rétention de décharge d'environ 93 % ou plus, mesurée après 200 cycles, chaque cycle incluant une charge cc/cv à 0,5C et une décharge cc/cv à 0,5C à 2,5 à 4,2 V (coupure) et à une température de 45 °C.

10. Véhicule comprenant la batterie secondaire au lithium selon la revendication 7.
